# EUROPEAN PATENT APPLICATION

(11) **EP 2 537 497 A1**
(43) Date of publication of application: **26.12.2012**
(21) Application number: 11744714.4
(22) Date of filing: 17.02.2011
(51) Int. Cl.: A61F 13/49, A61F 13/53

(54) **DISPOSABLE WEARING ARTICLE**

(30) Priority: 19.02.2010 JP 2010035353
(71) Applicant: Unicharm Corporation, Ehime 799-0111 (JP)
(72) Inventor: TODA, Haruki, Kanonji-shi Kagawa 769-1602 (JP); NAKAJIMA, Kaiyo, Kanonji-shi Kagawa 769-1602 (JP)
(74) Representative: Murgatroyd, Susan Elizabeth
(86) International application number: PCT/JP2011/053412
(87) International publication number: WO 2011/102428

(57) **Abstract**

A disposable wearing article improved so that a pressure put on the wearer's body may be partially alleviated. A disposable urine absorbent pad 1 as one example of the disposable wearing articles has an inner sheet 17, an outer sheet 18 and a liquid-absorbent structure 20 interposed between these sheets. The liquid-absorbent structure 20 is formed on an absorbent surface 21 thereof with a pair of first raised portions 31 protruding to the side of the inner sheet 17. The first raised portions 31 lie in the rear waist region and extend in a longitudinal direction Y along lateral edges 25 of the liquid-absorbent structure 20. The first raised portions 31 may be formed of the same material as a core 23 of the liquid-absorbent structure 20, specifically, of a mixture of fluff pulp and superabsorbent polymer particles.

## Description

### {Technical Field}

The present invention relates to disposable wearing articles and more particularly to disposable wearing articles including a unique liquid-absorbent structure such as disposable urine absorption pads, disposable diapers, disposable toilet-training pants, disposable incontinent pants and disposable sanitary pants.

### {Background}

Conventionally, it is known in a diaper having front and rear waist regions and a crotch region extending between these front and rear waist regions to attach high bulk members to regions contactable with the wearer's sacral bone. For example, JP 2006-101895 A (PTL1) discloses a diaper having an inner sheet, an outer sheet, an absorbent structure interposed between these sheets and high-bulk members interposed between this absorbent structure the inner sheet. The high-bulk members are provided in a sacral bone covering region overlapping the wearer's sacral bone when the diaper is put on the wearer's body so that a cushion is formed between the diaper's sacral bone and the bed.

### {Citation List}

### {Patent Literature}

{PTL 1}: JP 2006-101895 A

### {Summary of Invention}

### {Technical Problem}

In the diaper disclosed in PTL 1, the high-bulk members are formed in the sacral bone covering regions and so that the diaper may be always kept in contact with the sacral bone. The sacral bone is protuberant from the remaining region and therefore the sacral bone is compressed against the side of the bed under the wearer's own body weight, setting off development of bedsores. Contact of the diaper with the region of the wearer's body in which the bedsores have developed increases irritation to the wearer's skin and may retard healing of the bedsores.

An object of this invention is to provide a disposable wearing article improved so that a pressure put on the wearer's body may be partially alleviated.

### {Solution to Problem)

This invention relates to a disposable wearing article having a longitudinal direction and a lateral direction and including a chassis and a liquid-absorbent structure. The chassis includes a skin-facing side facing the wearer's body and a non-skin-facing side opposite to the skin-facing side, front and rear waist regions and a crotch region extending between the front and rear waist regions so that these regions are contiguous to each other in the longitudinal direction, front and rear ends extending in the transverse direction and lateral edges extending in the longitudinal direction. The liquid-absorbent structure is located at least in the crotch region and the rear waist region and has an absorbent surface and a bottom surface.

In the present invention, the liquid-absorbent structure is formed on at least one of the absorbent surface and the bottom surface with first raised portions extending across the rear waist region along the lateral edges, respectively.

According to one embodiment of this invention, the liquid-absorbent structure is formed on at least one of the absorbent surface and the bottom surface with a second raised portion in a midsection of the liquid-absorbent structure as viewed in the transverse direction so as to extend from the first raised portions toward the side of the crotch region.

According to another embodiment of this invention, the liquid-absorbent structure is formed between the first raised portions with a connecting raised portion adapted to connect the first raised portions to each other so as to be located on the side of the rear end.

According to still another embodiment of this invention, the liquid-absorbent structure includes liquid-absorbent core and a wrapping sheet adapted to wrap the core and at least one of the first, second and connecting raised portions is formed of the same material as the core.

According to further another embodiment of this invention, at least one of the first, second and connecting raised portions is formed of macromolecular compound.

According to yet another embodiment of this invention, the macromolecular compound is foamed polyurethane.

According to one alternative embodiment of this invention, a skin-contactable sheet is attached to the skin-facing side of the chassis along opposite lateral margins extending in the longitudinal direction so that the skin-contactable sheet may overlap at least one of the first and second raised portions.

### {Advantageous Effects of Invention}

According to this invention, particularly according to one or more embodiments thereof, the liquid-absorbent structure is formed in the rear waist region with the first raised portions protruding from at least one of the absorbent surface and the bottom surface so that these first raised portions extend in the longitudinal direction along the lateral edges, respectively. Consequently, the depressed portion extending in the longitudinal direction is defined in the vicinity of the transverse middle section of the liquid-absorbent structure. In view of the fact that the sacral bone lies in the vicinity of the transverse middle region of the wearer's body, the liquid-absorbent structure should not come in direct contact with the sacral bone and sensibly compress this sacral bone when the wearing article according to this invention is put on the wearer's body. Thus, it is possible to alleviate the skin irritation due to the friction between the liquid-absorbent structure and the wearer's skin.

### {Brief Description of Drawings}

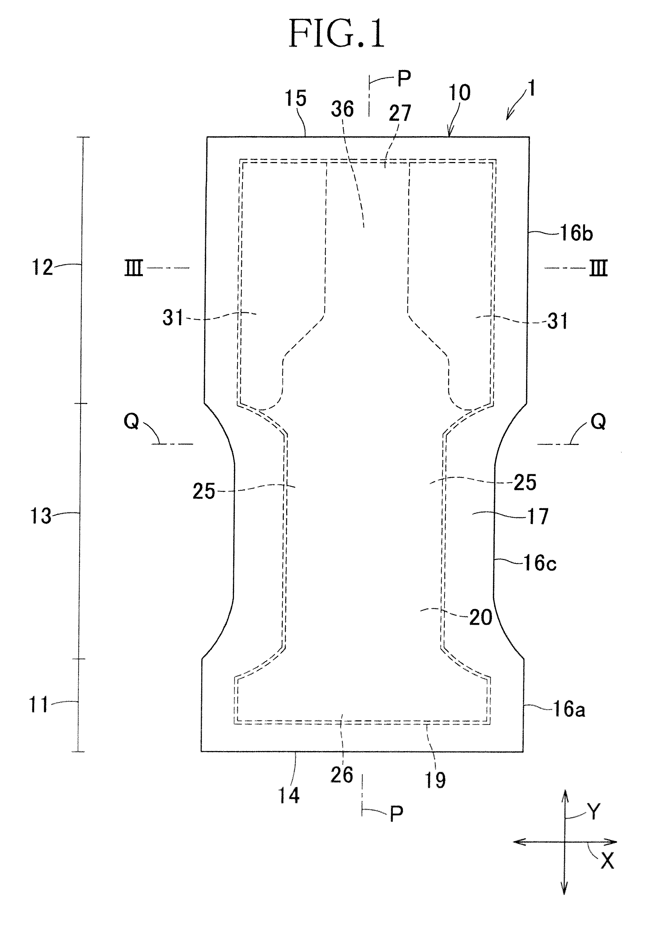

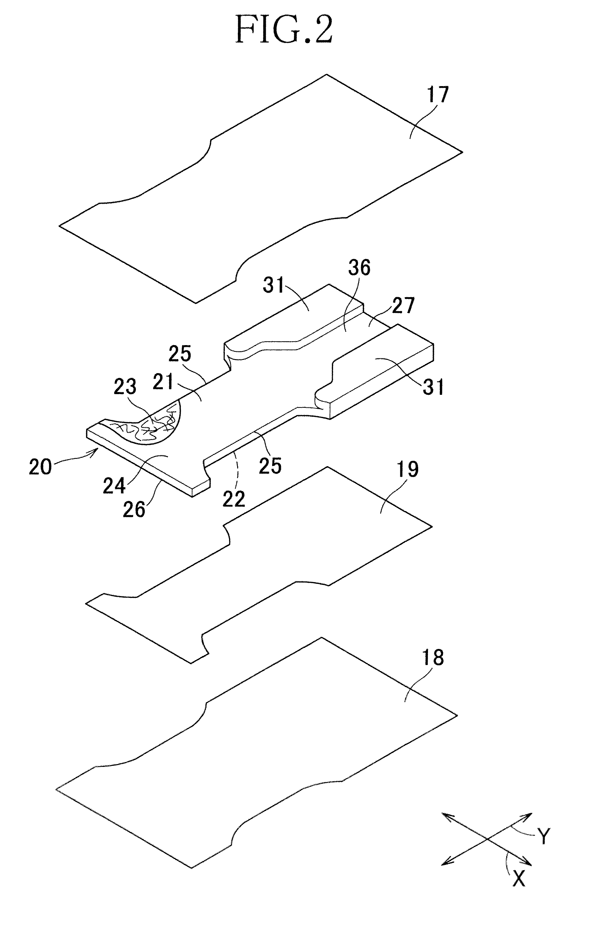

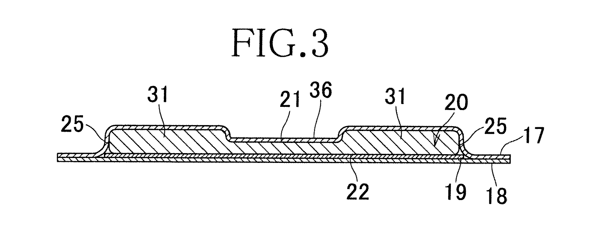

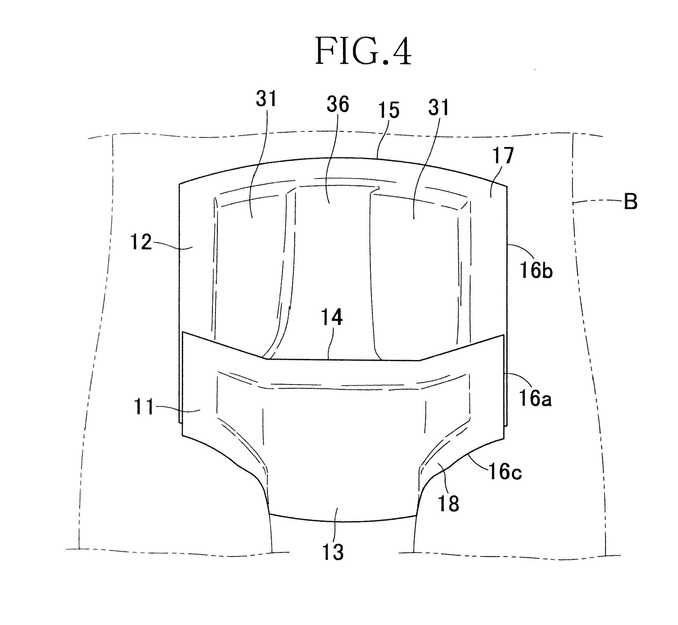

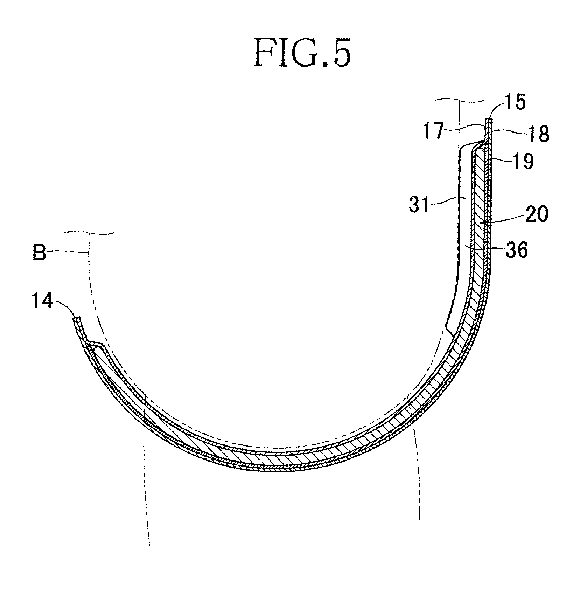

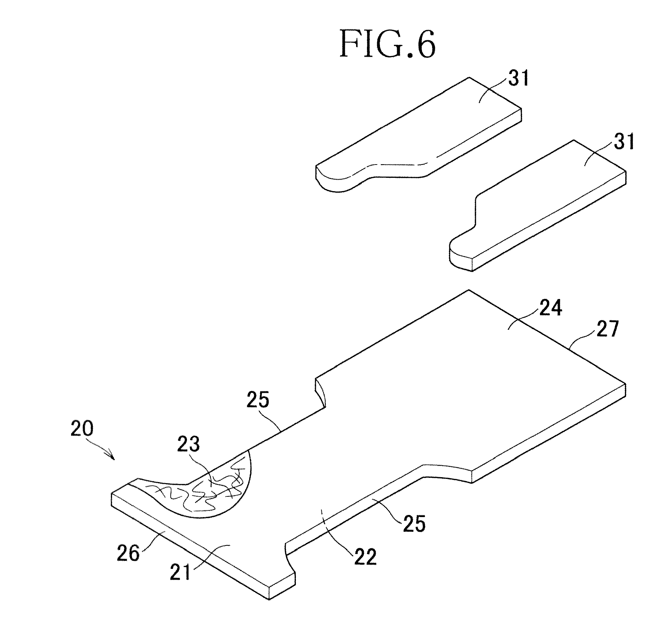

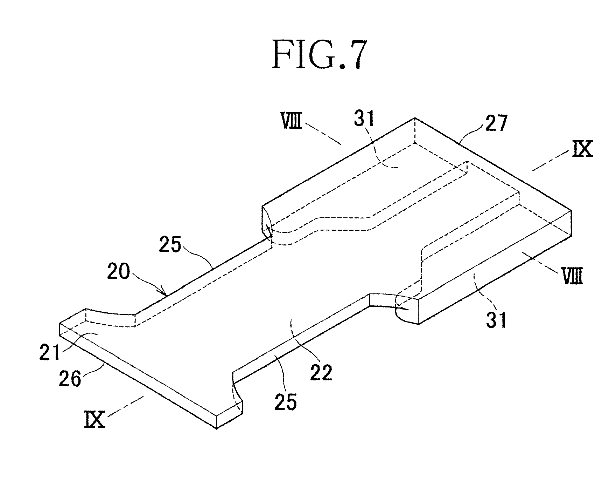

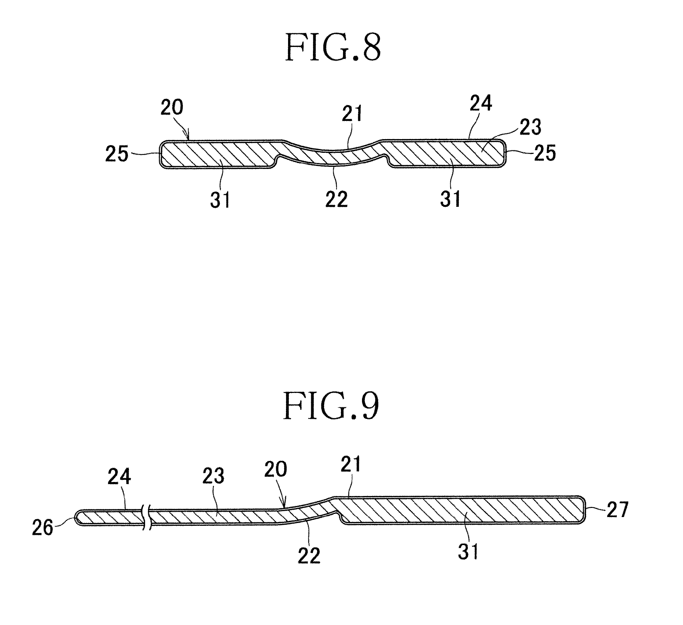

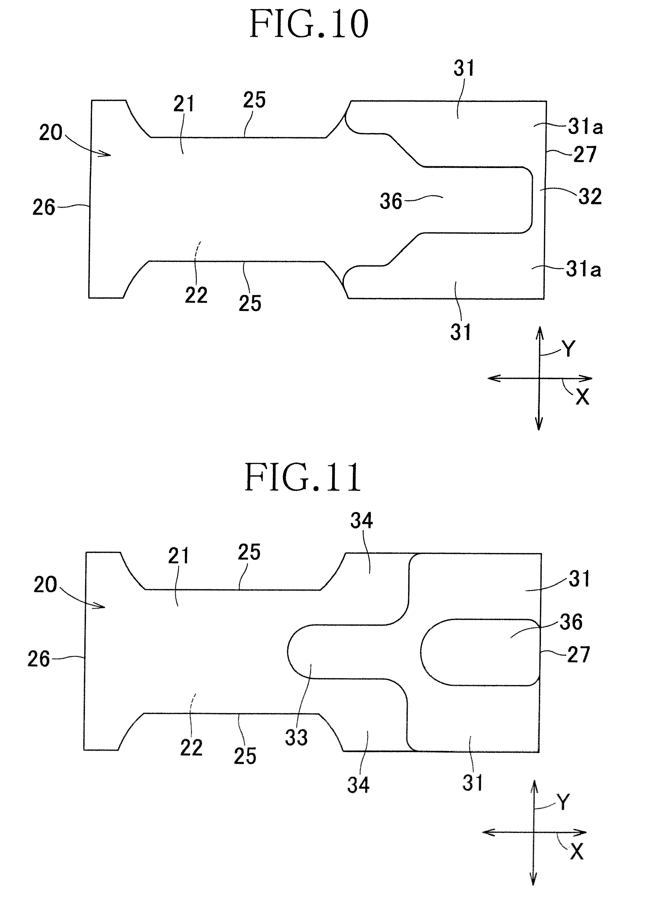

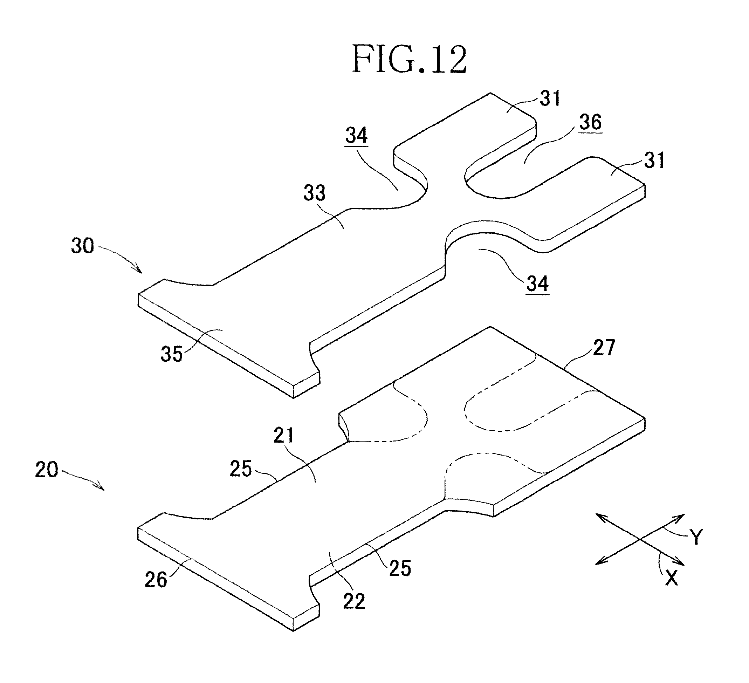

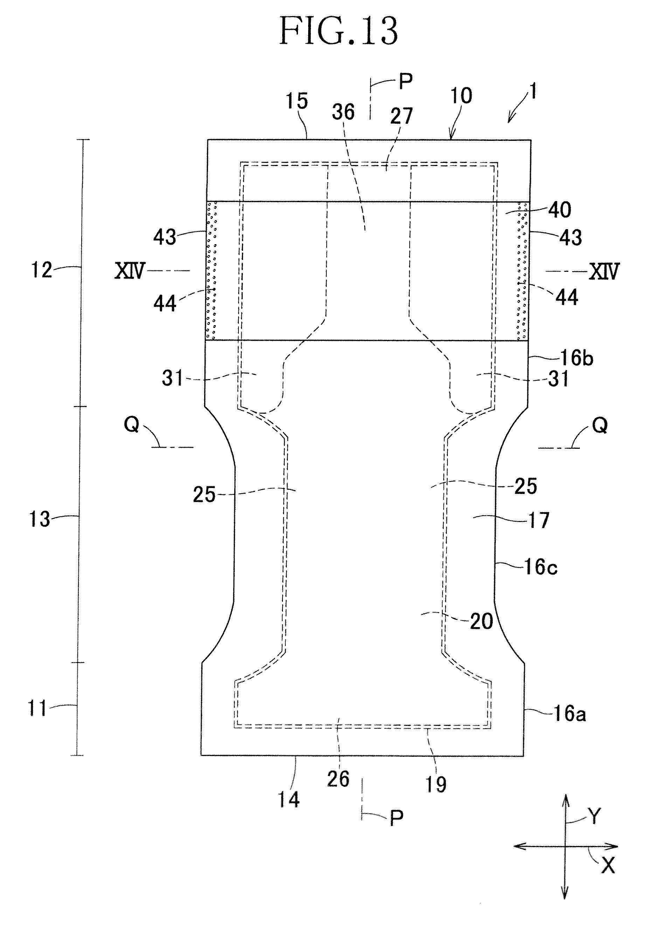

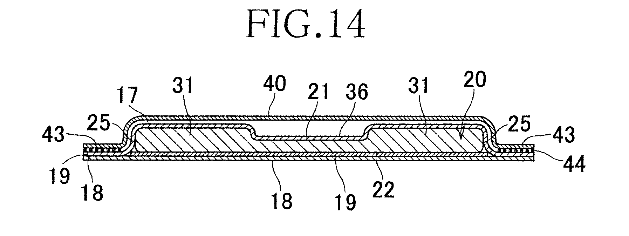

{Fig. 1} Fig. 1 is a plan view of a disposable urine absorbent pad as one example of disposable wearing articles according to a first embodiment.
{Fig. 2} Fig. 2 is an exploded view corresponding to Fig. 1.
{Fig. 3} Fig. 3 is a sectional view taken along line III-III in Fig. 1.
{Fig. 4} Fig. 4 is a diagram illustrating the pad put on the wearer's body.
{Fig. 5} Fig. 5 is a diagram illustrating the pad put on the wearer's body.
{Fig. 6} Fig. 6 is an exploded view of a liquid-absorbent structure according to a second embodiment.
{Fig. 7} Fig. 7 is a perspective view of the liquid-absorbent structure according to a third embodiment.
{Fig. 8} Fig. 8 is a sectional view taken along line VIII-VIII in Fig. 7.
{Fig. 9} Fig. 9 is a sectional view taken along line IX-IX in Fig. 7.
{Fig. 10} Fig. 10 is a plan view of the liquid-absorbent structure according to a fourth embodiment.
{Fig. 11} Fig. 11 is a plan view of the liquid-absorbent structure according to a fifth embodiment.
{Fig. 12} Fig. 12 is a plan view of the liquid-absorbent structure according to a sixth embodiment.
{Fig. 13} Fig. 13 is a plan view of the pad according to a seventh embodiment.
{Fig. 14} Fig. 14 is a sectional view taken along line XIV-XIV in Fig. 13.

### {Description of Embodiments}

### <First Embodiment>

Fig. 1 is a plan view of a disposable urine absorbent pad 1 as one example of disposable wearing articles according to this invention. Fig. 2 is a partially cutaway exploded perspective view of the pad 1. Fig. 3 is a sectional view taken along line III-III in Fig. 1. Figs. 4 and 5 exemplarily illustrating the pad 1 put on the wearer's body. The pad 1 has an imaginary longitudinal center line P-P bisecting a dimension thereof in the transverse direction X and an imaginary transverse center line Q-Q bisecting a dimension thereof in the longitudinal direction Y wherein the pad 1 is substantially symmetric about the imaginary longitudinal center line P-P. In each paired symmetric portions, only one of these symmetric portions will be denoted by a reference sign and the other will not be reference signed as the case may be.

The pad 1 includes a chassis 10 and a panel-like liquid-absorbent structure 20 wherein the chassis 10 has a skin-facing side facing the wearer's body, a non-skin-facing side opposite thereto and facing the wearer's garment, a front waist region 11, a rear waist region 12 and a crotch region 13 extending between the front and rear waist regions 11, 12, and the liquid-absorbent structure 20 extending across the crotch region 13 into the front and rear waist regions 11, 12.

The chassis 10 has front and rear ends 14, 15 opposite to each other about the imaginary transverse center line Q-Q and extending in the transverse direction X and lateral edges opposite to each other about the imaginary longitudinal center line P-P and extending in the longitudinal direction Y. The lateral edges include front lateral edges 16a extending in the front waist region 11, rear lateral edges 16b extending in the rear waist region 12 and crotch lateral edges 16c extending in the crotch region 13. The front and rear lateral edges 16a, 16b extend substantially in parallel to the imaginary longitudinal center line P-P and the crotch lateral edges 16c are concavely curved so as to be put in close contact with the wearer's thighs with high fit.

The chassis 10 includes an inner sheet 17 defining the skin-facing side, and an outer sheet 18 defining the side opposite thereto and facing the wearer's garment. Between these inner and outer sheets 17, 18, the liquid-absorbent structure 20 is interposed. The liquid-absorbent structure 20 has an absorbent surface 21 facing the inner sheet 17 and a bottom surface 22 facing the outer sheet 18. Between the bottom surface 22 and the outer sheet 18, a leakage-barrier film 19 adapted to cover the bottom surface 22 is interposed. The inner sheet 17 and the liquid-absorbent structure 20, the liquid-absorbent structure 20 and the leakage-barrier film 19, the leakage-barrier film 19 and the outer sheet 18 are respectively bonded to each other with hot melt adhesives or the like applied in an intermittent pattern.

As material for the inner and outer sheets 17, 18, a spun bonded fibrous nonwoven fabric, a spun bonded/melt blown/spun bonded (SMS) fibrous nonwoven fabric, an air-through nonwoven fabric or the like may be used. As material for the leakage-barrier film 19, a liquid-impervious and a moisture-pervious plastic film may be used. The liquid-absorbent structure 20 may be obtained by compression molding a mixture of fluff pulp and superabsorbent polymer particles or other materials to form a liquid-absorbent core 23 and then wrapping this liquid-absorbent core 23 with a liquid-diffusive wrapping sheet 24.

The liquid-absorbent structure 20 extends across at least the crotch region 13 and further extends in the longitudinal direction Y into the front and rear waist regions 11, 12. A dimension of the liquid-absorbent structure 20 in the transverse direction X is reduced in the crotch region 13 so as to be constricted toward the imaginary transverse center line Q-Q. In this embodiment, a large proportion of the crotch region 13 lies forward of the imaginary transverse center line Q-Q in the longitudinal direction Y and a dimension of the rear waist region 12 in the longitudinal direction Y is larger than that of the front waist region 11.

The liquid-absorbent structure 20 is formed on the absorbent surface 21 with a pair of first raised portions 31 which are protuberant toward the side of the inner sheet 17. The first raised portions 31 are located in the rear waist region 11 and extend in the longitudinal direction Y along lateral edges 25 of the liquid-absorbent structure 20. The first raised portions 31 are formed of the same material as that for the core 23 of the liquid absorbent structure 20, i.e., a mixture of fluff pulp and superabsorbent polymer particles and has a dimension in its thickness direction in a range of about 8 mm to about 30 mm.

The aforementioned first raised portions 31 and liquid-absorbent structure 20 may be integrally formed by depositing the material for the core 23 into a forming die. Specifically, the recess of the forming die for the liquid-absorbent structure 20 may be additionally provided with recesses for the first raised portions 31, then the material for the core 23 may be deposited in the forming die and compressed to form the first raised portions 31 and the liquid-absorbent structure 20. In this way, the first raised portions 31 and the liquid-absorbent structure 20 may be formed at once.

The above-mentioned liquid-absorbent structure 20 may be obtained by a compression molding technique. Thus the core 23 in the liquid-absorbent structure 20 may have a mass per unit area, for example, of about 200 g/m² and the core 23 in respective regions in which the respective first raised portions overlap the liquid-absorbent structure 20 may have a mass per unit area, for example, in a range of about 600 to about 800 g/m². By differentiating the mass per unit area to a range of about 400 to about 600 g/m², it is possible to dimension the thickness of the liquid-absorbent structure 20 so that the regions thereof formed with the first raised portions 31 may be about 8 mm thicker than the region thereof not formed with any raised portion. In this embodiment, the paired first raised portions 31 are spaced apart from each other by about 100 mm. It should be appreciated here that the mass per unit area of the core is not limited to the above-mentioned range but may be appropriately varied within the range usually adopted in the relevant technical field.

Figs. 4 and 5 illustrate the pad put on the wearer's body wherein the wearer B is indicated by imaginary lines. As illustrated, the crotch region 13 is placed on the wearer's crotch, the front waist region 11 is placed on the wearer's ventral side and the rear waist region 12 is placed on the wearer's dorsal side. Generally, the pad 1 is used in a manner such that the pad 1 is covered with the diaper or the diaper cover in order to keep the pad 1 in close contact with the wearer. In this regard, referring to Figs. 4 and 5, such a diaper or a diaper cover is not illustrated. According to this embodiment, the front lateral edges 16a and the rear lateral edges 16b of the pad 1 are not brought in contact with each other but spaced away from each other between the wearer's ventral side and the wearer's dorsal side.

In the pad 1, the rear waist region 12 is dimensioned in the longitudinal direction Y to be larger than that of the front waist region 11 and, in consequence, the wearer's dorsal side may be covered with the pad 1 over a sufficiently large area. Specifically the rear waist region 12 preferably has a dimension sufficient to cover a sacral bone region of the wearer B. The pad 1 is a slip-on article and suitable for the wearer who is obliged to lie on a bed most all day. In such pad 1, when the wearer is lying on the back, urine discharged by the wearer flows toward the dorsal side and such urine flowing toward the dorsal side may be reliably absorbed by dimensioning the length of the rear waist region 12 in the longitudinal direction Y to be sufficiently large.

The pad 1 is formed with the first raised portions 31 and, with the pad 1 put on the body of the wearer B, the first raised portions 31 come in contact with the body of the wearer B. The respective first raised portions 31 are provided to the liquid-absorbent structure 20 only along the opposite sides 25 thereof so that a depressed portion 36 may be defined between these first raised portions 31. In consequence, with the pad 1 put on the body of the wearer B, the sacral bone lying substantially on the center line of the body of the wearer B may be located on this depressed portion 36. Even when the wearer B lies on the back, the wearer' s own body weight scarcely impinges on the sacral bone region and therefore development of bedsores may be restricted. Even if the bedsores develop, the wearer's skin and the pad 1 should not perceptively friction each other and therefore it is possible to alleviate undesirable skin irritation.

The first raised portions 31 are formed so as to extend in the longitudinal direction Y along the lateral edges 25 of the liquid-absorbent structure 20 and therefore the depressed portion 36 defined between these raised portions 31 correspondingly extends in the longitudinal direction Y. Even if the sacral bone and the pad 1 are displaced relative to each other in the longitudinal direction Y when the wearer turns up an upper part of the bed and uses this upper part as a backrest, the sacral bone may be caught by the depressed portion 36 and the sacral bone should not come in contact with the remaining portion of the pad 1. In this way, undesirable skin irritation due to contact between the wearer's body and the pad 1 may be restricted even in such a situation.

In the regions in which the first raised portions 31 overlap the liquid-absorbent structure 20, the mass per unit area of the core is larger than that in the remaining region and therefore compression resistance/compressive elasticity may be made correspondingly higher in these overlapping regions than in the remaining region. Consequently, the first raised portions 31 would not be easily flattened out even under a pressure so as to maintain the shape of the depressed portion 36 defined between the paired first raised portions 31. In addition, the first raised portions 31 formed along the lateral edges may function as barrier walls adapted to prevent body waste such as urine from leaking out in the transverse direction X.

While the first raised portions 31 and the liquid-absorbent structure 20 are integrally formed according to this embodiment, it is also possible to form them separately and then to stack the first raised portions 31 on the liquid-absorbent structure 20. In this case, the first raised portions 31 may be formed on the outer side of the wrapping sheet 24 for the liquid-absorbent structure 20 or may be formed on the inner side of the wrapping sheet 24. It is also possible to compress the first raised portions 31 under a pressure higher than a pressure under which the liquid-absorbent structure 20 is compressed so that the mass per unit area of the core material in the first raised portions 31 may be higher than that of the core in the liquid-absorbent structure 20. In this case, the compression resistance of the first raised portions 31 may be sufficiently enhanced to prevent the first raised portions 31 from being deformed.

When the first raised portions 31 are formed of the core, it is also possible to mix thermoplastic fibers in this core. In the course of forming the first raised portions 31 from the core containing such thermoplastic fibers under the effect of compression and heating, the thermoplastic fibers are partially fusion-bonded together to form a net-like structure adapted to present fluff pulp and/or superabsorbent polymer particles from falling off. The net-like structure formed of the thermoplastic fibers may be expected also to improve the compressive elasticity of the first raised portions 31.

### <Second Embodiment>

Fig. 6 exemplarily illustrates the liquid-absorbent structure 20 used for the pad 1 according to a second embodiment. The components similar to those in the first embodiment will be denoted by the similar reference signs and details thereof will not be repeatedly described hereunder.

The liquid-absorbent structure 20 is formed of the liquid-absorbent core 23 and the liquid-diffusive wrapping sheet 24 adapted to wrap this core 23. The first raised portions 31 are formed of foamed polyurethane as one example of macromolecular material and bonded to the wrapping sheet 24 with hot melt adhesives (not shown). Use of water-repellent foamed polyurethane as material for the first raised portions 31 makes it possible to prevent body waste such as urine from leaking and oozing out in the transverse direction X. Additionally, use of the foamed polyurethane of which both the compression resistance and the compressive elasticity are high makes it possible to alleviate undesirable skin irritation which would otherwise be experienced by the wearer's skin due to contact with the first raised portions and to prevent the first raised portions 31 from being deformed even under the wearer's body weight.

The macromolecular material is not limited to the foamed polyurethane and it is also possible to use cushion material formed of polyester/polyolefin conjugate fibers, for example, "elk ™" manufactured by TEIJIN FIBERS, LTD. in Japan or "miracle Fiber ™" manufactured by Daiwabo Co., Ltd. in Japan. Use of such cushion material makes it possible to improve the breathability and to prevent development of stuffiness within the pad 1.

### <Third Embodiment>

Figs. 7 through 9 exemplarily illustrate a third embodiment of this invention wherein Fig. 7 is a perspective view of the liquid-absorbent structure 20, Fig. 8 is a sectional view taken along line VIII-VIII in Fig. 7 and Fig. 9 is a sectional view taken along line IX-IX in Fig. 7. This embodiment is characterized in that the first raised portions 31 are formed on the bottom surface 22 of the liquid-absorbent structure 20. The other constituents are similar to those in the first embodiment and these constituents similar to those in the first embodiment will be denoted by the reference signs similar to those in the first embodiments and details of these similar constituents will not be repeatedly described hereunder.

The first raised portions 31 are formed on the bottom surface 22 of the liquid-absorbent structure 20 so as to extend along the lateral edges 25. These first raised portions 31 are located in the rear waist region 12 and formed of the same material as the core 23 of the liquid-absorbent structure 20. With such a pad 1 put on the wearer's body, the regions of the liquid-absorbent structure 20 in which the first raised portions overlap the bottom surface 22 of the liquid-absorbent structure 20 come in relatively tight contact with the wearer's skin and the region of the liquid-absorbent structure 20 not overlapping the first raised portions 31 in which the first raised portions comes in relatively gentle contact with the wearer's skin. In the situation where a midsection in the transverse direction X of the liquid-absorbent structure 20 not overlapping the first raised portions 31 is put in gentle contact with the wearer's skin, the liquid-absorbent structure 20 may absorb voided body waste such as urine more quickly than in the situation where such midsection is out of contact with the wearer's skin. In consequence, body waste such as urine should not move along the wearer's skin and therefore leakage thereof due to such movements along wearer's skin may be prevented.

Between the first portions 31 in the transverse direction. X, liquid-absorbent structure 20 concavely sags toward the of the bottom surface 22 the effect of its own weight or a body weight of the wearer (See Fig. 8) . Also at the end which is adjacent to the crotch region 13, the liquid-absorbent structure 20 concavely sags toward the side of the bottom surface 22 (See Fig. 9). Such sagging of the liquid-absorbent structure 20 at a gentle angle may alleviate a discomfort feeling which would otherwise be experienced by the wearer due to the presence of the first raised portions 31. The first raised portions 31 may be formed from the mixture of fluff pulp and superabsorbent polymer particles or the like or from the suitable macromolecular compound. The constituents of this embodiment may be combined with the constituents of the other embodiments.

### <Fourth Embodiment>

Fig. 10 exemplarily illustrates the liquid-absorbent structure 20 according to a fourth embodiment This embodiment is characterized in that a connecting raised portion 32 is formed between the first raised portions 31 as viewed in the transverse direction X. The other constituents are similar to those in the first embodiment and these constituents similar to those in the first embodiment will be denoted by the reference signs similar to those in first embodiment and details of these similar constituents will not be repeatedly described hereunder.

The connecting raised portion 32 servers to connect respective rear ends 31a of the first raised portions 31 in the transverse direction X and formed on the absorbent surface 21 of the liquid-absorbent structure 20 in the similar fashion to the first raised portions 31. In this way, a wall extending in the transverse direction X extending in the transverse direction X is formed between the paired first raised portions 31 and, even if urine or the like accumulates in the depressed portion 36 defined between the paired first raised portions 31, it is possible to prevent such urine or the like from flowing out beyond the respective rear ends 31a of the paired first raised portions 31. The wearer's body weight is exerted on the first raised portions 31 and the connecting raised portion 32 and the influence of the body weight is more effectively dispersed than the case in which the body weight is exerted on the first raised portions 31 not connected by the connecting raised portion 32. In this way, deformation of the first raised portions 31 may be restricted. When the connecting raised portion 32 and the first raised portions 31 are formed from the same material, these raised portions 32, 32 may be integrally formed. Lit is also possible to form at least one of the first raised portions 31 and the connecting raised portion 32 from the macromolecular material. The constituents of this embodiment may be appropriately combined with the constituents of the other embodiments.

### <Fifth Embodiment>

Fig. 11 exemplarily illustrates the liquid-absorbent structure 20 according to a fifth embodiment characterized in that the liquid-absorbent structure 20 is formed in the middle in the transverse direction X with a second raised portion 33. The other constituents are similar to those in the first embodiment and these constituents similar to those in the first embodiment will be denoted by the reference signs similar to those in the first embodiment and details of these similar constituents will not be repeatedly described hereunder.

In the rear waist region, the liquid-absorbent structure 20 is formed with the pair of the first raised portions 31 extending along the lateral edges 25, respectively. The second raised portion 33 is formed contiguously to the first raised portions 31 in the longitudinal direction Y so as to extend from the rear waist region 12 to the crotch region 13. The second raised portion 33 is located substantially in the midsection in the transverse direction and in the longitudinal direction Y so that a substantially Y-shaped region may be defined by the first raised portions 31 and the second raised portion 33. These first raised portions and the second raised portion 33 are formed on the absorbent surface 21 of the liquid-absorbent structure 20.

Between the rear waist region 12 and the crotch region 13, the first raised portions 31 cooperate with the second raised portion 33 to define a pair of depressed portions 34 along the lateral edges 25 of the liquid-absorbent structure 20. With the pad 1 put on the wearer's body, these depressed portions 34 are preferably located on the ischial bone region of the wearer. Both the sacral bone region and the ischial bone region are protuberant relative to the remaining region of the wearer's body and particularly the ischial bone region is the region being apt to come in contact with a chair or a bed when the wearer sits thereon. Thus, there is a possibility that bedsores might develop and/or the wearer's skin might be sensibly irritated due to such contact in the ischial bone region also. However, the depressed portions 34 formed so as to overlap this ischial bone region may restrict development of the bedsores and alleviate the skin irritation. In addition, the side of a front end 26 of the portion 36 defined the paired first portions 31 is blocked by the second raised portion 33 and, in consequence, it is possible to prevent urine or the like voided into the depressed portion 36 from moving toward the side of the front end 26. The first raised portions and the second raised portion may be formed from the mixture of fluff pulp and superabsorbent polymer particles or the like or may be formed from the macromolecular compound. The constituents in this embodiment may be combined with the constituents in each of the first through fourth embodiments.

### <Sixth Embodiment>

Fig. 12 exemplarily illustrates the liquid-absorbent structure according to a sixth embodiment. This embodiment is characterized in that the liquid-absorbent structure 20 is formed with the first raised portions 31, the second raised portion 33 and a third raised portion 35. The other constituents are similar to those in the first embodiment and these constituents similar to those in the first embodiment will be denoted by the reference signs similar to those in the first embodiment and details of these similar constituents will not be repeatedly described hereunder.

The first portions 31 are formed in rear waist region 12 so as to along the lateral edges of liquid-absorbent structure 20. The second raised portion 33 is formed so as to be located in the midsection of the liquid-absorbent structure 20 in contiguity with the first raised portions 31 in the longitudinal direction Y and to extend from the rear waist region 12 to the crotch region 13. The third raised portion 35 is formed in the front waist region 11 in contiguity with the second raised portion 33 in the longitudinal direction Y so that the third raised portion 35 may almost entirely overlap the liquid-absorbent structure 20 in the front waist region 11. Having been formed with the first through third raised portions 31, 33, 35, a lamination layer 30 adapted to be laminated on the liquid-absorbent structure 20 is obtained.

Formation of the first through third raised portions 31, 33, 35 makes it possible to define the depressed portion 36 between the paired first raised portions 31 in the transverse direction and to define the depressed portions 34 between the respective first raised portions 31 and the second raised portion 33. In this way, undesirable irritation to the wearer's sacral bone and ischial bone be alleviated. in addition, the first through third raised portions 31, 33, 35 almost entirely come in contact with the wearer's skin and make it possible to enlarge the contact area correspondingly to dispense the influence of the wearer's body weight. The first through third raised portions 31, 33, 35 may be formed from the mixture of fluff pulp and superabsorbent polymer particles or the like, or from the macromolecular compound. The constituents in this embodiment may be combined with the constituents in each of the first through fifth embodiments.

### <Seventh Embodiment>

Figs. 13 and 14 exemplarily illustrate a seventh embodiment characterized in that a skin-contactable sheet 40 is attached to the liquid-absorbent structure in a region thereof corresponding to the first raised portions 31. The other constituents are similar to those in the first embodiment and these constituents similar to those in the first embodiment will be denoted by the reference signs similar to those in the first embodiment and details of these similar constituents will not be repeatedly described hereunder.

The liquid-absorbent structure 20 is formed on the absorbent surface 21 thereof with the first raised portions 31. The respective first raised portions 31 lie in the rear waist region 12 and extend in the longitudinal direction Y along the lateral edges 25 of the liquid-absorbent structure 20. The inner sheet 17 is laminated on the first raised portions 31 and a skin-contactable sheet 40 is attached to the inner side of the inner sheet 17. More specifically, the skin-contactable sheet 40 extends between one of the rear lateral edges 16b and the other of the rear lateral edges 16b and is bonded only along opposite lateral margins thereof to the inner sheet 17 by bonding means 44 such as hot melt adhesives. In other words, a region of the skin-contactable sheet 40 defined between the opposite bonding means 44 is not bonded to the inner sheet 17. As material for such skin-contactable sheet 40, the material which is at low risk of frictioning and irritating the wearer's skin, for example, a spun bonded fibrous nonwoven fabric or an SMS fibrous nonwoven fabric may be used.

The skin-contactable sheet 40 is bonded to the inner sheet 17 only along the opposite lateral margins 43 so that the region defined between these bonded lateral margins may be spaced from the inner sheet 17 and, in consequence, the skin-contactable sheet 40 may move relative to the inner sheet 17.

With the pad 1 put on the wearer's body, the first raised portions 31 and the skin-contactable sheet 40 located on the wearer's dorsal side, preferably on the region corresponding to the sacral bone. When, in this state, the wearer turns up the upper part of the bed to sit up, the wearer's buttocks may be displaced forward under the effect of the wearer's own body weight. Even in this case, the skin-contactable sheet 40 and the inner sheet 17 move relative to each other in the longitudinal direction Y or in the transverse direction X as if these sheets pass each other and therefore the skin-contactable sheet 40 may conform to movements of the wearer's dorsal side.

The relative movements of the skin-contactable sheet 40 and the inner sheet 17 as has been described above prevents undesirable skin irritation from developing due to the friction between the wearer's skin and the skin-contactable sheet 40 and makes it possible to restrict development of the bedsores further reliably. Even when the first raised portions 31 come in relatively close contact with the wearer's skin, the skin irritation due to such close contact may be alleviated since no sensible friction occurs. In conseguence, a discomfort feeling experienced by the wearer due to formation of the first raised portions 31 may be also alleviated.

As first raised portions 31, those according to the first through sixth ments may be selectively used and second, third and connecting raised portions may be also formed in this seventh embodiment.

It is possible to make the skin-contactable sheet 40 elastically contractible in the longitudinal direction Y or in the transverse direction X. In this way, the skin-contactable sheet 40 may further smoothly conform to movements of the wearer's body. To make the skin-contactable sheet 40 elastically contractible, appropriate elastic members may be attached to the skin-contactable sheet 40 or an elastically stretchable and contractible nonwoven fabric or the like may be used as material for the skin-contactable sheet 40.

While the first through seventh embodiment have been described on the based on the pad adapted to be placed on the inner side of the diaper in actual use, this invention is applicable also to the other wearing articles such as disposable diapers. Rewarding the diaper, this invention is applicable not only to pull-on type diapers of which the front waist lateral edges and the rear lateral edges are previously joined together but also to open-type diapers of which the front lateral edges and the rear lateral edges are not previously joined together.

While the disposable wearing article in which the crotch region 13 having the dimension in the transverse direction X dimensioned to be smaller than those of the front and rear waist region has been described hereinbefore, a substantially rectangular wearing article in which the dimension in the transverse direction X is nearly uniform in the front waist region 11, the rear waist region 12 and the crotch region 13 is not excluded from this invention. In such a rectangular wearing article, the portion adapted to lie in the wearer's crotch may be defined as the crotch region 13, the portion adapted to lie on the wearer's ventral side extending forward may be defined as the front waist region 11 and the portion adapted to lie on the dorsal side extending rearward from the crotch region 13 may be defined as the rear waist region 12. In this case, the first raised portions 31 are preferably located closer to the side of the rear end than the imaginary transverse center line Q-Q.

The components of the pad 1 are not limited to those described in the specification but the other various type of known materials wifely used in the relevant technical field may be used without limitation.

In the aforementioned embodiments, one of these embodiments may be added with or combined with the components and/or configurations of other embodiments. For example, the skin-contactable sheet 40 used in the seventh embodiment may be applied to the other embodiments.

### {Reference Signs List}

1 disposable urine absorbent pad (disposable wearing article)
10 chassis
11 front waist region
12 rear waist region
13 crotch region
14 front end
15 rear end
16b rear lateral edges
20 liquid-absorbent structure
21 absorbent surface
22 bottom surface
23 core
24 wrapping sheet
31 first raised portions
32 connecting region
33 second raised portion
35 third raised portion
40 skin-contactable sheet
43 opposite lateral margins
44 bonding means

## Claims

1. A disposable wearing article having a longitudinal direction and a lateral direction, including:
a chassis including a skin-facing side facing the wearer's body and a non-skin-facing side opposite thereto, front and rear waist regions and a crotch region extending between the front and rear waist regions so that these regions are contiguous to each other in the longitudinal direction, front and rear ends extending in the transverse direction and lateral edges extending in the longitudinal direction; and
a liquid-absorbent structure located at least in the crotch region and the rear waist region and having an absorbent surface and a bottom surface, wherein:
the liquid-absorbent structure is formed on at least one of the absorbent surface and the bottom surface with first raised portions extending across the rear waist region along the lateral edges, respectively.

2. The disposable wearing article defined by claim 1, therein the liquid-absorbent structure is formed on at least one of the absorbent surface and bottom surface with a second raised portion in a midsection of liquid-absorbent structure as viewed in the transverse direction so as to extend from the first raised portions toward the side of the crotch region.

3. The disposable wearing article defined by claim 1 or 2, wherein the liquid-absorbent structure is formed between the first raised portions with a connecting raised portion adapted to connect the first raised portions to each other so as to be located on the side of the rear end.

4. The disposable wearing article defined by any one of claims 1 through 3, wherein the liquid-absorbent structure includes liquid-absorbent core and a wrapping sheet adapted to wrap the core and wherein at least one of the first, second and connecting raised portions is formed of the same material as the core.

5. The disposable wearing article defined by any one of claims 1 through 4, wherein at least one of the first, second and connecting raised portions is formed of macromolecular compound.

6. The disposable wearing article defined by claim 5, wherein the macromolecular compound is foamed polyurethane.

7. The disposable wearing article defined by any one of claims 1 through 6, wherein a skin-contactable sheet is attached to the skin-facing side of the chassis along opposite lateral margins extending in the longitudinal direction so that the skin-contactable sheet may overlap at least one of the first and second raised portions.
